(19) Europäisches Patentamt
European Patent Office
Office européen des brevets

(11) **EP 3 313 496 B1**

## (12) EUROPEAN PATENT SPECIFICATION

(45) Date of publication and mention
of the grant of the patent:
**11.11.2020 Bulletin 2020/46**

(51) Int Cl.:
**A61M 25/00** *(2006.01)*        **A61B 5/00** *(2006.01)*
**A61B 5/02** *(2006.01)*        **A61B 5/0215** *(2006.01)*
**A61M 25/09** *(2006.01)*

(21) Application number: **16815059.7**

(22) Date of filing: **14.06.2016**

(86) International application number:
**PCT/US2016/037389**

(87) International publication number:
**WO 2016/209665 (29.12.2016 Gazette 2016/52)**

(54) **APPARATUS FOR INTRAVASCULAR MEASUREMENTS**

VORRICHTUNG FÜR INTRAVASKULÄRE MESSUNGEN

APPAREIL POUR MESURES INTRAVASCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.06.2015 US 201514747692
02.11.2015 US 201514930168**

(43) Date of publication of application:
**02.05.2018 Bulletin 2018/18**

(73) Proprietor: **Zurich Medical Corporation
St. Paul, MN 55112 (US)**

(72) Inventors:
 • **SHAM, Kin-Joe
  St. Paul, Minnesota 55112 (US)**

 • **DONADIO, James V., III.
  St. Paul, Minnesota 55112 (US)**
 • **CHAN, Charles C.H.
  St. Paul, Minnesota 55112 (US)**

(74) Representative: **Roberts, David
Page White & Farrer
Bedford House
John Street
London WC1N 2BF (GB)**

(56) References cited:
**US-A- 4 884 579        US-A- 5 308 324
US-A1- 2008 140 101        US-A1- 2013 218 032
US-A1- 2013 296 722        US-A1- 2014 081 244
US-A1- 2015 005 648        US-B2- 7 628 763
US-B2- 8 551 021**

## Description

FIELD OF THE INVENTION

The disclosed technology relates to intravascular diagnosis. More particularly, the disclosed technology relates to diagnosing the severity of stenosis in the vasculature of a patient.

BACKGROUND

Reduced blood flow due to atherosclerotic occlusion of vessels is a major cause of vascular diseases. Pressure measurements in arterial vessels and particularly in coronary arteries prior to treatment have been used for lesion characterization and treatment selection. More specifically, pressure gradient across a lesion has been clinically used as an indicator for lesion severity. Measurements made during and after treatment allow one to assess therapy efficacy. Existing equipment for monitoring intravascular measurements have multiple, separate parts and bulky monitors. There is, accordingly, continuing interest in improved monitoring equipment. US2015/005648A1 discloses a diagnosis apparatus and method. In one aspect of the disclosed technology, a intravascular diagnosis apparatus includes a monitoring guidewire and a display unit. The monitoring guidewire includes a core wire and a sensor disposed in a distal region of the core wire. US2013/218032A1 discloses a pressure guidewire comprising: a shaft tube with a proximal section; a middle section extending from the proximal section of the shaft tube, the middle section having greater flexibility than the proximal section; an inner hypotube installed substantially within the middle section for optimal mechanical properties; a pressure sensor with a communication means routed through the middle section and the proximal section; and a sensor housing for receiving the pressure sensor.

SUMMARY

The disclosed technology relates to diagnosing the severity of stenoses in the vasculature of a patient. The invention is defined by appended claims 1-6.

In one aspect of the disclosed technology, a monitoring guidewire for a medical procedure includes a core wire having a first length, wherein the core wire has a diameter of at most 0.018cm (0.007 inches), a pressure sensor disposed in a distal region of the core wire, and a hypotube surrounding the core wire and having a second length that is less than the first length, the hypotube having laser etching along at most a 40cm length of a distal portion of the hypotube, wherein the hypotube has an outer diameter between 0.033 and 0.036cm (0.013 and 0.014 inches); and wherein the core wire comprises at least one of: MP35N, L605, Elgiloy, or an alloy including nickel, cobalt, molybdenum and chromium; and wherein the hypotube comprises at least one of: polyemide; nitinol; stainless steel; nylon; polyurethane; PTFE.

In one embodiment, the core wire has a length of approximately 180 cm. In one embodiment, the hypotube has a length of approximately 177 cm.

In one embodiment, the laser etching is configured to provide the hypotube with a torque response that approximates a torque response of a 0.036cm (0.014 inch) workhorse guidewire.

In one embodiment, the hypotube has an inner diameter less than 0.028cm (0.011 inches).

In one embodiment the hypotube is more flexible than the core wire for at most the length of the distal portion of the hypotube having the laser etching.

These aspects and embodiments of the disclosed technology are exemplary and do not limit the scope of the disclosed technology, which will be apparent from a reading of the following detailed description and the associated drawings.

BRIEF DESCRIPTION OF THE DRAWINGS

FIG. 1 is a block diagram of an exemplary intravascular diagnosis apparatus in accordance with the disclosed technology.

FIG. 2 is a block diagram of an embodiment of the disclosed technology;

FIG. 3 is a diagram of an exemplary apparatus in accordance with the disclosed technology;

FIG. 4 is another diagram of an exemplary apparatus in accordance with the disclosed technology;

FIG. 5 is a diagram of an exemplary distal tip of the disclosed monitoring guidewire;

FIG. 6 is a diagram of two embodiments of the distal tip of the disclosed monitoring guidewire;

FIG. 7 is a diagram of one position for the disclosed monitoring guidewire for estimating fractional flow reserve;

FIG. 8 is a diagram of another position for the disclosed monitoring guidewire for estimating fractional flow reserve;

FIG. 9 is a flow diagram of exemplary operation of the disclosed technology for computing simultaneous fractional flow reserve;

FIG. 10 is a flow diagram of exemplary operation of the disclosed technology for computing push-forward fractional flow reserve;

FIG. 11 is a flow diagram of exemplary operation of the disclosed technology for computing pull-back fractional flow reserve;

FIG. 12 is a diagram of a perspective view and a cross-sectional view of one embodiment of the disclosed monitoring guidewire;

FIG. 13 is a diagram of a perspective view and a cross-sectional view of another embodiment of the disclosed monitoring guidewire;

FIG. 14 is a diagram of a perspective view and a cross-sectional view of yet another embodiment of the disclosed monitoring guidewire; and

FIG. 15 is a diagram of exemplary characteristics of the disclosed monitoring guidewire.

DETAILED DESCRIPTION

The disclosed technology relates to diagnosing the severity of stenosis in the vasculature of a patient. The disclosed technology can be used as an adjunct to conventional angiographic procedures to provide important quantitative measurements of a blood vessel lumen.

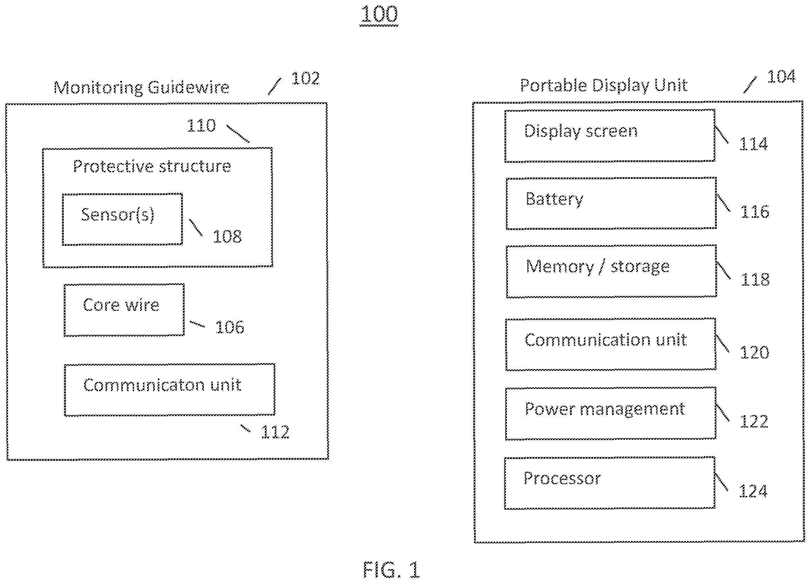

Referring now to FIG. 1, there is shown a block diagram of an exemplary intravascular diagnosis apparatus in accordance with the disclosed technology. The illustrated apparatus 100 includes a monitoring guidewire 102 and a portable display unit 104. In one example, the portable display unit 104 can be a handheld display unit, such that any and all aspects and embodiments described herein as being applicable to a portable display unit are also applicable to the disclosed handheld display unit. In one example, the handheld display unit can be equal to or less than 30cm x 30 cm x 30cm in size. In operation, the monitoring guidewire 102 is introduced into the vasculature of a patient with the assistance of conventional interventional equipment known to those skilled in the art, such as catheters. The portable display unit 104 can communicate with the monitoring guidewire 102 and can display information based on the communications received from the monitoring guidewire 102.

The illustrated monitoring guidewire 102 can include several components, including a core wire 106 and one or more sensors 108 disposed in a distal region of the core wire 106. As used herein, the terms "distal" and "proximal" refer to physical directions within a blood vessel lumen. Specifically, in relation to the insertion point of a device into a patient, the term "distal" refers to the direction from the insertion point inwards into a blood vessel, and the term "proximal" refers to the direction from the inside of a blood vessel out towards the insertion point. As used herein, the terms "proximal" and "distal" can also refer to different ends of a device, with "proximal" being the end towards an insertion point into a blood vessel lumen and with "distal" being the end away from the insertion point.

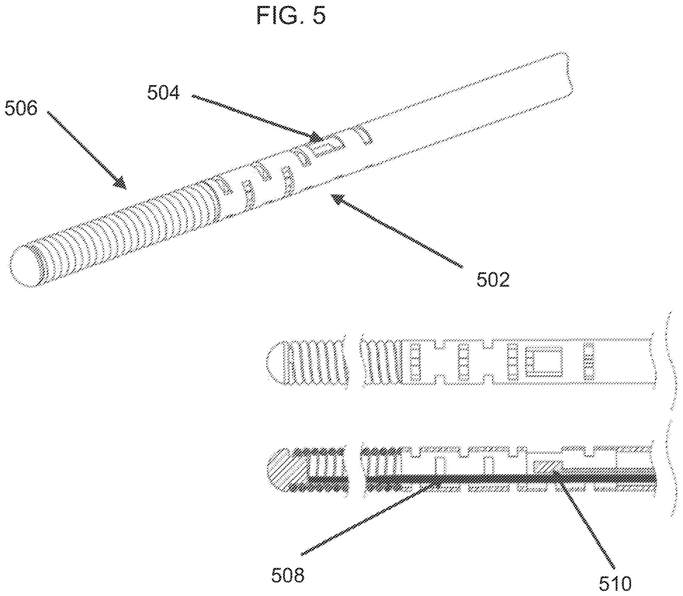

With continuing reference to FIG. 1, the one or more sensors 108 disposed in a distal region of the core wire 106 can include one or more hemodynamic pressure sensors and/or one or more temperature sensors. In one example, the pressure sensor(s) can be a piezo-resistive pressure sensor. As illustrated in FIG. 1, the monitoring guidewire 102 can also include a protective structure 110 surrounding the sensor(s) 108, and can include a communication unit 112. The protective structure 110 of the monitoring guidewire 102 will be described in more detail later herein in connection with FIGS. 5-6.

In one example, the communication unit 112 can employ wireless communication technology such as bluetooth, WiFi (802.11), or any other wireless technology. In one example, the communication unit 112 can be a wireline communication unit that can include one or more wires for communicating electromagnetic signals and/or one or more optical fibers for communicating optical signals. The monitoring guidewire 102 can include other components that are not illustrated, such as a power source, A/D converters, application specific integrated circuits (ASIC), a processor, memory, timing circuitry, and/or other power, analog, or digital circuitry. Such components will be known to those skilled in the art.

Referring now to the illustrated portable display unit 104, the portable display unit 104 can include a display screen 114, one or more batteries 116, memory and/or storage 118, a communication unit 120, power management unit 122, and a processor 124. In one embodiment, the processor 124 can be a general purpose processor or can be an application specific integrated circuit. In one embodiment, the display screen 114 can be a liquid crystal display, an organic light emitting diode display, or another type of display technology. In one example, the memory / storage 118 can include one or more of solid state memory / storage, magnetic disc storage, and/or any other type of memory / storage that will be known to those skilled in the art. In one example, the memory / storage 118 can include software instructions that are executed by the processor 124. In one example, the communication unit 120 can employ wireless communication technology such as bluetooth, WiFi (802.11), or any other wireless technology. In one example, the communication unit 120 can be a wireline communication unit that can include one or more wires for communicating electromagnetic signals and/or one or more optical fibers for communicating optical signals. The portable display unit 104 can include other components that are not illustrated, such as user interface, operating system software, display driver circuitry, A/D converters, application specific integrated circuits (ASIC), timing circuitry, and/or other power, analog, or digital circuitry. Such components will be known to those skilled in the art.

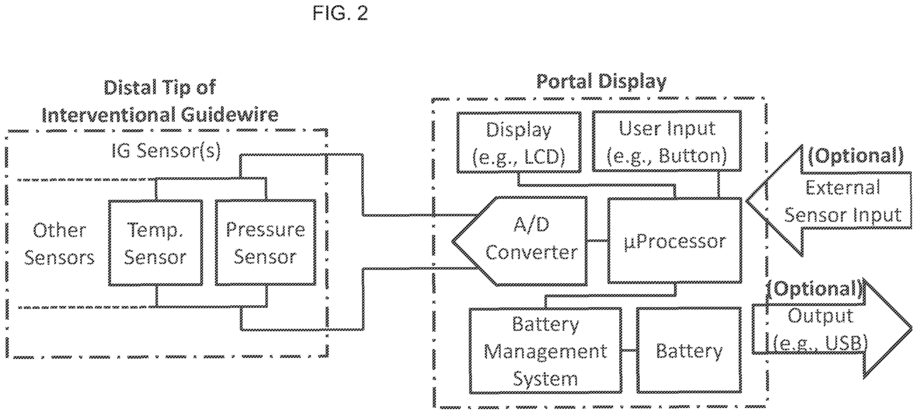

Referring now to FIG. 2, there is shown a system block diagram of another example of the disclosed technology. The monitoring guidewire contains a pressure sensor and/or other sensors at the distal end. The electrical signals from the sensor(s) can be sent over a wire connection to the portable display unit. The portable display unit can include a communications port that receives external sensor input such as aortic output pressure (AO IN) from pressure transducers / hemodynamic systems (not shown). The portable display unit can also include an output communication port for outputting data to an external storage device, to another display, to a printer, and/or to a hemodynamic system (not shown).

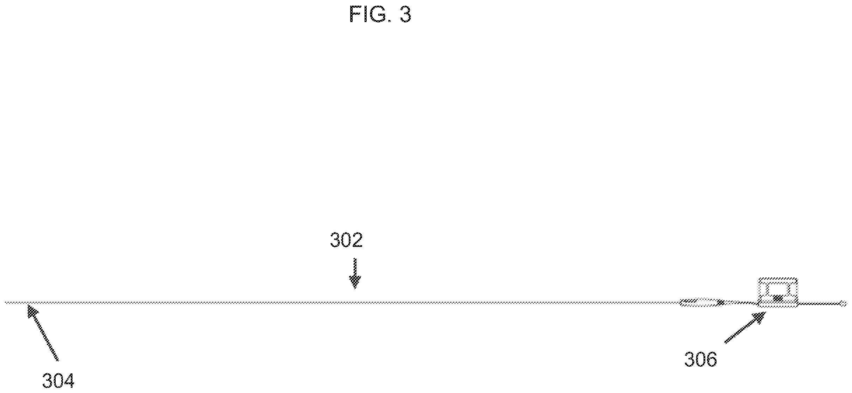

Referring now to FIG. 3, there is shown an exemplary embodiment of the disclosed intravascular diagnosis apparatus. In one example, the monitoring guidewire 302 can be approximately 180 centimeters in length. In other examples, the monitoring guidewire 302 can be another length. The monitoring guidewire 302 can have one or more sensors in the distal region 304 of the monitoring guidewire 302. In the illustrated embodiment, the portable display unit 306 can have a small form factor such that it is a handheld display unit. In one example, a handheld display unit can be equal to or less than 30cm x 30 cm x 30cm in size.

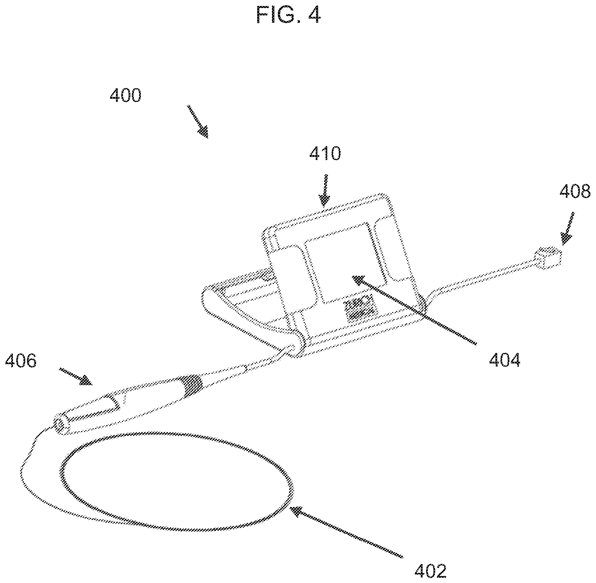

FIG. 4 is a diagram of another exemplary embodiment of the disclosed intravascular diagnosis apparatus. In the illustrated example, the monitoring guidewire 402 can be attached and detached from a connector 406 of the portable display unit 400. In one example, the connector 406 can include a button (not shown) which opens an aperture in the connector 406. To attach or detach the monitoring guidewire 402, a user can press and hold the button of the connector 406 and insert the monitoring guidewire 402 into the aperture until the monitoring guidewire 402 is fully inserted into connector 406. Once inserted, the user can release the button, which will then secure the monitoring guidewire 402 in place and provide a connection between the monitoring guidewire 402 and connector 406. In other examples, the connector 406 can engage the monitoring guidewire 402 by a screw engagement, a twist engagement, a snap engagement, or an interference fit.

In one example, the connector connection establishes a communicative connection between the monitoring guidewire 402 and the portable display unit 400. The monitoring guidewire 402 and the connector 406 can contain electrical wires that connect the monitoring guidewire 402 to the portable display unit 400 and convey signals from the monitoring guidewire sensor(s) to the portable display unit 400.

In one example, the connector connection establishes a mechanical connection between the monitoring guidewire 402 and the connector 406 to control the guidewire 402 within a vasculature. In the illustrated example, the connector 406 is tethered to the main housing 410 of the portable display unit 400. In one example, the tether can be 6 inches to 12 inches long and can allow a user to manipulate the monitoring guidewire 402 freely without the portable display unit main housing 410 being an impediment. In one example, the tether can be another length. In one example (not shown), the connector can be a connection port integrated in the portable display unit main housing 410.

In one example, the connector 406 establishes a communicative connection with the monitoring guidewire 402. In one example, a torquer (not shown) can be configured to engage the monitoring guidewire 402 to control the guidewire within a vasculature when the monitoring guidewire 402 is not mechanically and/or electrically connected to the connector 406. In one example, the torquer can be configured to engage the monitoring guidewire 402 to control the guidewire within a vasculature when the monitoring guidewire 402 is mechanically and/or electrically connected to the connector 406. In one embodiment, the monitoring guidewire 402 does not need a torquer or the connector 406 for insertion into the vasculature of a patient and for navigation therein, and provides this capability by itself.

With continuing reference to FIG. 4, the portable display unit 400 includes a display screen 404 that can display sensor measurements and/or computed information (e.g., fractional flow reserve ratio), in numerical format and/or in waveform format. The portable display unit 400 can include one or more buttons (not shown) or a touch screen to allow a user to provide input to the portable display unit 400. In one embodiment, the screen 404 of the portable display unit can be folded in the main housing 410 before use to minimize the size of packaging when delivering the portal display unit 400. When a user takes the portable display unit 400 out of the packaging for use, the user can pivot the screen 404 from the folded position to an open position (as illustrated), providing an appropriate viewing angle to the user for the diagnosis procedure. In one example, pivoting of the display screen 404 from the folded position to an open position acts as an ON switch that enables power to be delivered to the portable display unit.

In the illustrated example, the portable display unit 400 also includes a communication port 408. In one embodiment, the communication port 408 allows a user to connect the portable display unit 400 to an external system (not shown). The external system can communicate a sensor signal to the portable display unit 400 through the communication port 408. In one example, the sensor signal received at the communication port can be can be a pressure measurement and can be used in calculating fractional flow reserve.

Referring again to FIG. 1, the monitoring guidewire 102 can include a protective structure 110 surrounding the sensor(s) 108. With reference to FIG. 5, there is shown a diagram of an exemplary protective structure 502 surrounding the sensor(s) 510 at the distal region of the monitoring guidewire. In the illustrated example, the protective structure 502 is a housing that has been laser etched with a particular pattern cut to provide flexibility and/or torque translation at the distal tip or portion of the monitoring guidewire where the sensor 510 resides. The sensor(s) 510 can be situated in the laser etched housing at a window 504 in the housing so as to allow blood to contact the sensor(s) 510 in order to take sensor measurements. In the illustrated example, the core wire 508 can be grinded to provide an appropriate profile for balancing flexibility and torque translation. In one example, the monitoring guidewire need not include a core wire 508. Rather, the protective structure 502 can extend along the entire monitoring guidewire or a substantial portion thereof, and can be laser etched along some or all portions to provide desired flexibility and/or torque translation.

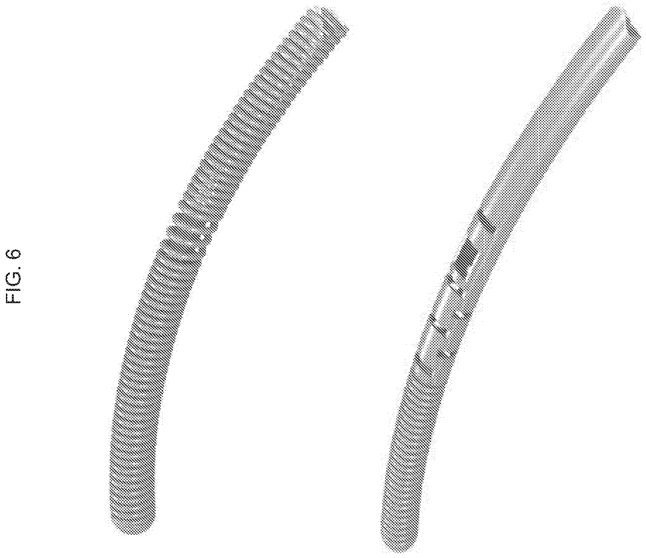

Referring to FIG. 6, there is shown a diagram of two exemplary protective structures surrounding the sensor(s) at the distal region of a monitoring guidewire.

One of the examples is a laser etched housing as described in connection with FIG. 5. The other example provides a coil over the sensor(s) as the protective structure. The coil is relaxed to create a window where the sensor(s) are located to allow blood to contact the sensor(s).

The following description will now reference FIG. 1, and in particular, the battery 116 and the power management unit 122 of the portable display unit 104. In one aspect of the disclosed technology, the portable display unit 104 can be configured to operate for a predetermined duration or for a predetermined number of uses, and then be disposed. The battery 116 and/or power management unit 122 can implement these features so that the portable display unit 104 can be inoperable after being used for a particular duration or for a particular number of diagnosis procedures. Even so, the portable display unit 104 can be disposed while it is still operable, prior to it being inoperable.

In one example, the predetermined duration can correspond to the approximate length of time of a single intravascular diagnosis procedure. In one example, the predetermined duration can correspond to the approximate length of time of multiple diagnosis procedures, such as three procedures. In one example, the predetermined duration can be twelve hours or twenty-four hours or several days. In one aspect of the disclosed technology, the portable display unit 104 can include one or more batteries 116 that are configured to power the portable display unit 104 for the desired duration, such that the batteries 116 are substantially depleted at the end of the desired duration. In one example, the one or more batteries 116 are non-rechargeable, so that the portable display unit 104 is disposed after the batteries 116 are depleted. In one example, the power management unit 122 can control the operating time of the portable display unit 104 by preventing the portably display unit 104 from powering down after the display screen 114 is turned on. In such an embodiment, the portable display unit 104 will operate continuously until the batteries 116 are depleted or substantially depleted. The portable display unit 104 can be disposed prior to the batteries 116 being depleted, while the portable display unit 104 is still operable.

In one example, the portable display unit 104 can track the number of diagnosis procedures performed and can be configured to be inoperable after a particular number of procedures has been performed. In one example, the portable display unit 104 can track the number of diagnosis procedures performed by the number of times the portable display unit 114 has been turned on and/or off. In one example, the portable display unit 104 can be configured to be inoperable after a single diagnosis procedure has been performed. In one aspect of the disclosed technology, the power management unit 122 can prevent the portable display unit 104 from being powered on after the particular number of procedures has been reached. The batteries 116 can be rechargeable and can be recharged by a power source of the portable display unit 104 and/or by a power source external to the portable display unit 104. Even when the batteries 116 are not yet depleted, the power management unit 122 can cause the portable display unit 104 to be inoperable by preventing the batteries 116 from powering the portable display unit 104.

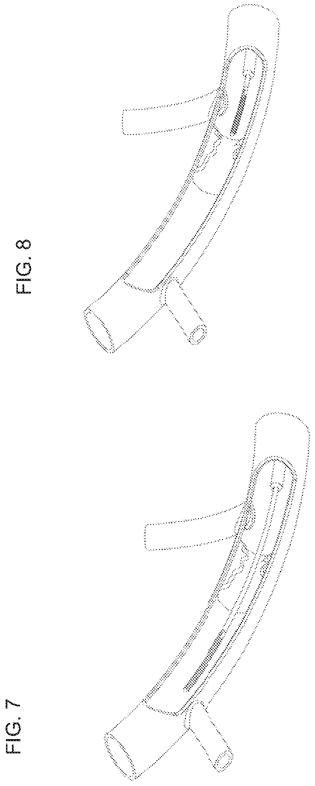

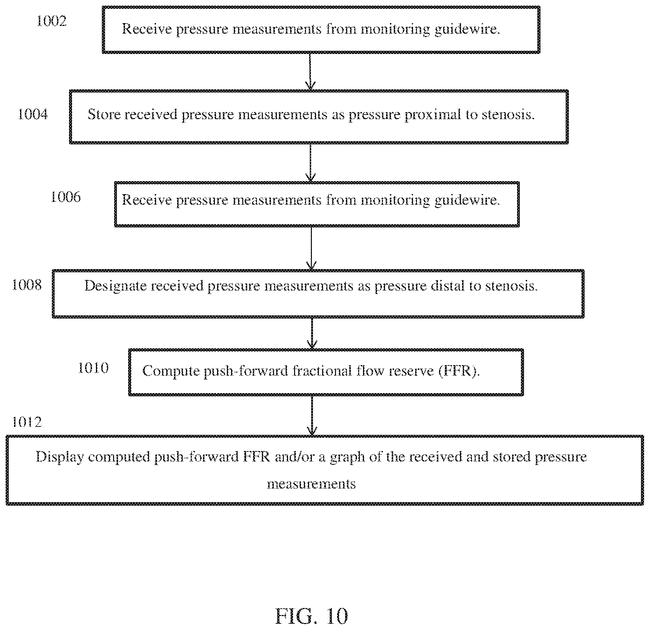

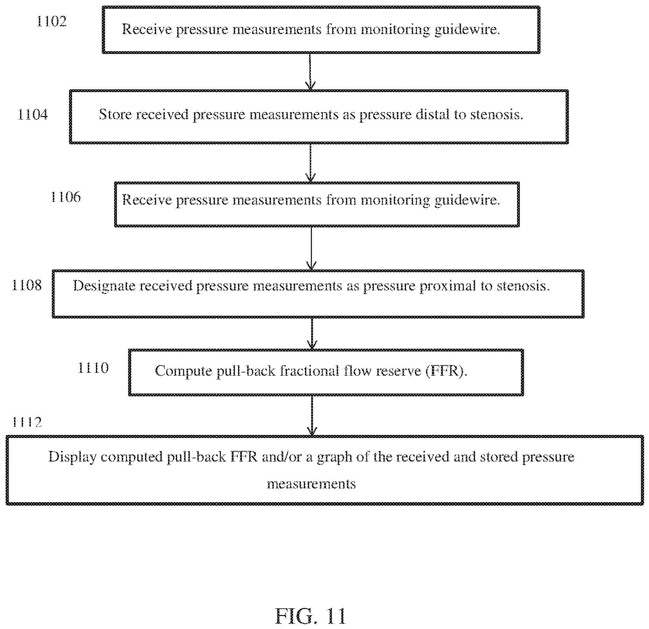

The intravascular diagnosis procedure will now be described with continuing reference to FIG. 1 and with reference to FIGS. 7-11. Diagnosing the severity of one or more stenoses within the vasculature of a patient has been studied based on hemodynamic pressure measurements distal to a stenosis in comparison with aortic output pressure. The ratio of pressure distal to a stenosis to the aortic output pressure is known as "factional flow reserve", or FFR. The value of the FFR indicates the severity of the stenosis, and clinical data provides guidance on the type of surgical procedure that would be effective for particular FFR ranges.

The disclosed technology includes multiple ways of computing FFR, including what will be referred to herein as "push-forward FFR", "pull-back FFR", and "simultaneous FFR". Each of these can be implemented by software code or machine code stored in memory / storage 118 of the portable display unit 104 (FIG. 1). The processor 124 can execute the software code to compute the FFR, and the resulting information can be displayed on the display screen 114. Each of the computation methods will now be described.

Simultaneous Fraction Flow Reserve

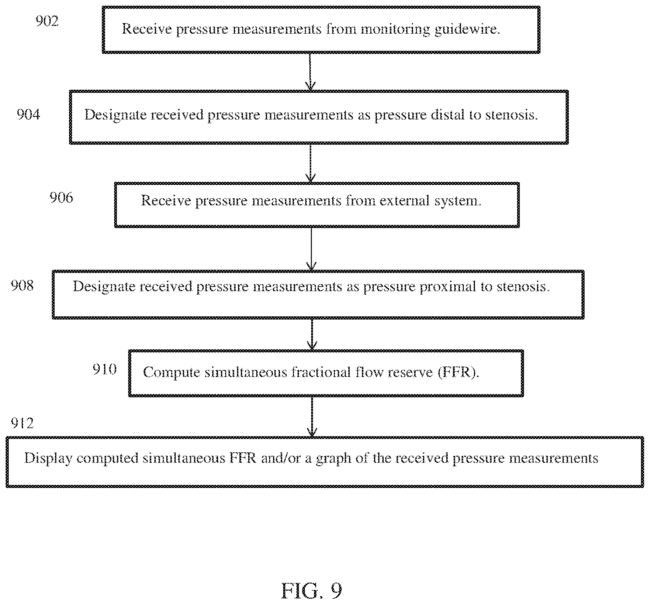

Simultaneous FFR involves simultaneous pressure readings from two separate pressure sensors, and a computation of FFR in real-time as the pressure readings from the two separate pressure sensors are received. Referring to FIG. 1 and FIG. 9, one pressure sensor is located in the monitoring guidewire 102, and is used to measure pressure distal to a stenosis in a patient. The pressure readings can be communicated by the communication unit 112 of the monitoring guidewire 102 to the communication unit 120 of the portable display unit 104

(902). This communication can be a wireless communication or can be a wireline communication through, for example, the connector illustrated in FIG. 3. The other pressure sensor can measure aortic output pressure and is external to the apparatus 100 of FIG. 1. The portable display unit 104 can designate the received pressure measurements as pressure distal to a stenosis (904). The external sensor readings can be communicated to the communication unit 120 of the portable display unit by, for example, the communication port illustrated in FIG. 3 (906). The portable display unit 104 can designate the received pressure measurements as pressure proximal to a stenosis (908). The portable display unit 104 can compute the simultaneous FFR as the pressure measurements are received (910), by the formula:

$FFR = (P_{sensor}-P_{ra})/(P_{port}-P_{ra})$, where:

> $P_{port}$ are moving means over time of real-time pressure measurements received at the communications port,
>
> $P_{sensor}$ are moving means over time of real-time pressure measurements from the pressure sensor in the distal region of the core wire of the monitoring guidewire, and
>
> $P_{ra}$ is a constant, which can be zero or another constant value.

In one example, the moving means over time can compute the mean over a window of time that spans one heartbeat. In other embodiments, the window of time can span less than one heartbeat or more than one heartbeat. As new sensor measurements are received over time (902, 906), the window can include newer measurements and remove older measurements to compute the moving means.

The portable display unit 104 can receive pressure measurements and can compute the simultaneous FFR based on the received measurements. The portable display unit 104 can store the received pressure measurements and/or the computed simultaneous FFR in memory / storage 118, and can display the computed simultaneous FFR and/or a graph of the received pressure measurements on the display screen 114 (912).

Push-Forward Fractional Flow Reserve

In contrast to simultaneous FFR, the push-forward FFR does not receive external pressure measurements. With continuing reference to FIG. 1, push-forward FFR is computed using pressure measurements from only the pressure sensor(s) 108 in the distal region of the monitoring guidewire 102. Using traditional angiography, a stenosis can be located and, as shown in FIG. 8, the monitoring guidewire can be inserted into a patient to a point proximal to the stenosis. Pressure can be measured at this position by the sensor(s) 108 and communicated by the communication unit 112 to the portable display unit 104 (1002). The portable display unit 104 can store the measurements in this position in the memory / storage 118 as pressure proximal to a stenosis (1004). Next, the monitoring guidewire 102 can be pushed forward past the stenosis to a point distal to the stenosis, as illustrated in FIG. 7. Pressure can be measured at this position by the sensor(s) 108 and communicated by the communication unit 112 to the portable display unit 104 (1006). The portal display unit 104 can designate the pressure measurements received at this position as pressure distal to the stenosis (1008). The processor 124 can compute the push-forward FFR (1010) by the formula: $FFR = (P_{sensor}-P_{ra})/(P_{saved}-P_{ra})$, where:

> $P_{saved}$ are moving means over time of recorded pressure measurements proximal to the stenosis,

$P_{sensor}$ are moving means over time of real time pressure measurements distal to the stenosis, and
$P_{ra}$ is a constant, which can be zero or another constant value.

Aspects of computing the moving means over time were described above in connection with simultaneous FFR, and such aspects apply to push-forward FFR as well.

The portal display unit 104 can display the computed push-forward FFR and/or a graph of the received and stored pressure measurements (1012).

Push-forward FFR can be computed in the case of one stenosis and can also be computed in the case of multiple stenosis. In either case, $P_{saved}$ are moving means over time of pressure measurements proximal to all of the stenosis. In one embodiment, $P_{saved}$ are moving means over time computed based on recorded pressure measurements. In one example, $P_{saved}$ are moving means over time computed and recorded as pressure measurements are received, and the pressure measurements may or may not be recorded. For example, in the case of two stenoses, $P_{saved}$ are based on pressure measurements proximal to both the first and second stenosis. When the monitoring guidewire pressure sensor 108 is pushed forward to a position between the first and the second stenosis, $P_{sensor}$ are based on real time pressure measurements between the two stenoses. Push-forward FFR can be calculated in this position and displayed on the display screen 114. When the monitoring guidewire pressure sensor 108 is pushed forward to a position distal to both the first and second stenoses, $P_{sensor}$ are based on real time pressure measurements distal to both of the two stenoses. Push-forward FFR can be calculated in this position and displayed on the display screen 114. Thus, push-forward FFR enables FFR to be computed and displayed as the monitoring guidewire 102 is pushed forward across one or more stenoses in a blood vessel lumen. The only measurements and/or moving means that need to be recorded for push-forward FFR computations are pressure measurements and/or moving means of pressure measurements proximal to all stenoses, and this is performed at the outset.

Pull-back Fractional Flow Reserve

Similar to push-forward FFR, the pull-back FFR does not receive external pressure measurements. Rather, pull-back FFR is computed using pressure measurements from only the pressure sensor(s) 108 in the distal region of the monitoring guidewire 102. Using traditional angiography, a stenosis can be located and, as shown in FIG. 7, the monitoring guidewire can be inserted into a patient to a point distal to the stenosis. Pressure can be measured at this position by the sensor(s) 108 and communicated by the communication unit 112 to the portable display unit 104 (1102). The portable display unit 104 can store the measurements in this position in the memory / storage 118 as pressure distal to a stenosis (1104). Next, the monitoring guidewire 102 can be pulled back through the stenosis to a point proximal to the stenosis, as illustrated in FIG. 8. Pressure can be measured at this position by the sensor(s) 108 and communicated by the communication unit 112 to the portable display unit 104 (1106). The portable display unit 104 can designate the measurements received in this position as pressure proximal to a stenosis (1108). The processor 124 can compute the pull-back FFR (1110) by the formula:

$$FFR = (P_{saved}-P_{ra})/(P_{sensor}-P_{ra})$$

where:

$P_{saved}$ are moving means over time of recorded pressure measurements distal to the stenosis,
$P_{sensor}$ are moving means over time of real time pressure measurements proximal to the stenosis, and
$P_{ra}$ is a constant, which can be zero or another constant value.

Aspects of computing the moving means over time were described above in connection with simultaneous FFR, and such aspects apply to pull-back FFR as well.

The portal display unit 104 can display the computed pull-back FFR and/or a graph of the received and stored pressure measurements (1112).

Pull-back FFR can be computed in the case of one stenosis and can also be computed in the case of multiple stenosis. In either case, $P_{sensor}$ are based on real-time pressure measurements proximal to all of the stenosis, which are the final pressure measurements that are taken. For example, in the case of two stenoses, the monitoring guidewire pressure sensor 108 is initially placed at a position distal to both the first and the second stenoses. Pressure can be measured at this position by the sensor(s) 108 and communicated by the communication unit 112 to the portable display unit 104. In one embodiment, $P_{saved\_d1}$ are moving means over time computed later based on recorded pressure measurements. In one embodiment, $P_{saved\_d1}$ are moving means over time computed and recorded while the pressure measurements are received in this position, and the pressure measurements may or may not be recorded. The memory / storage 118 can record the pressure measurements in this position and/or computed moving means over time based on such pressure measurements. Pull-back FFR cannot yet be calculated because there is no real-time measurement yet proximal to all of the stenoses. Next, the monitoring guidewire 102 can be pulled back through the first stenosis to a point between the first and second stenosis. Pressure can be measured at this position by the sensor(s) 108 and communicated by the communication unit 112 to the portable display unit 104. In one example, $P_{saved\_d2}$ are moving means over time computed later based on recorded pressure measurements. In one example, $P_{saved\_d1}$ are moving means over time computed and recorded while the pressure measurements are received in this position, and the pressure measurements may or may not be recorded. The memory / storage 118 can record the pressure measurements in this position and/or computed moving means over time based on such pressure measurements. Once again, pull-back FFR cannot yet be calculated because there is no real-time measurement yet proximal to all of the stenoses. Lastly, the monitoring guidewire 102 can be pulled back through the second stenosis to a point proximal to both the first and second stenosis. Real-time pressure can be measured at this position by the sensor(s) 108 and communicated by the communication unit 112 to the portable display unit 104. Only at this point are there enough measurements to compute the two pull-back FFR: $FFR_1 = (P_{saved\_d1}-P_{ra})/(P_{sensor}-P_{ra})$ and $FFR_2 = (P_{saved\_d2}-P_{ra})/(P_{sensor}-P_{ra})$. Therefore, pull-back FFR does not allow FFR to be calculated and displayed as the monitoring guidewire is being pulled back through multiple stenoses.

Accordingly, three computations for fractional flow reserve have been described above in connection with FIGS. 7-11. In one aspect of the disclosed technology, and with reference to FIG. 1, the portable display unit 104 is configured with capability to compute fractional flow reserve using any of the three ways. In one example, the portable display unit 104 can be configured to automatically use one of the three ways of computing fractional flow reserve. In one example, the portable display unit 104 can be configured to automatically select one of the three ways of computing fractional flow reserve when a condition is present and to automatically select another of the three ways of computing fractional flow reserve when other conditions are present. In one example, the portable display unit 104 can be configured to permit a user to manually select one of the three ways of computing fraction flow reserve.

The disclosed technology measures pressure and calculates fractional flow reserve (FFR). FFR is a calculation that has been clinically demonstrated to assist in determining whether to treat or not to treat an intermediate coronary lesion. Using the disclosed technology will thus assist a physician in determining what to do with an intermediate lesion. The disclosed FFR equations are exemplary and do not limit the scope of the disclosed technology.

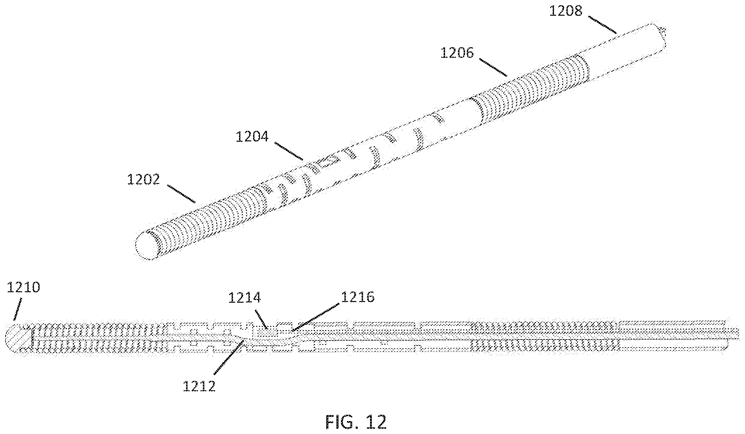

Referring again to FIG. 3 and FIG. 4, monitoring guidewires 302/402 in accordance with the disclosed technology have been described above herein. Various examples of the monitoring guidewire will now be described with respect to FIGs. 12-15.

FIG. 12 shows a perspective view and a cross-sectional view of one embodiment of a monitoring guidewire. The illustrated guidewire housing includes four segments: a distal coil 1202, a slotted tube 1204, an intermediate coil 1206, and a non-slotted proximal tube 1208. The slotted tube 1204 can include a slot pattern that is configured to provide desired properties and characteristics, such as flexibility and/or torque control. The illustrated pattern is merely exemplary and other patterns are contemplated to be within the scope of the disclosed technology. The distal coil 1202 and the intermediate coil 1206 can be configured to provide desired characteristics, such as torsion and/or compression. Referring to the cross-sectional view shown in FIG. 12, the illustrated monitoring guidewire includes an atraumatic tip 1210, a core wire 1212, one or more sensors 1214, and one or more signal wires 1216.

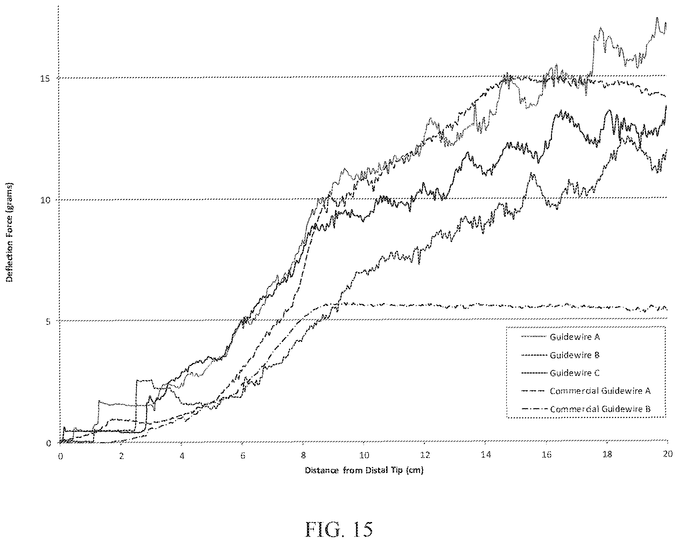

In one aspect of the disclosed technology, the illustrated monitoring guidewire can provide a torque response that approximates that of a 0.014 inch workhorse guidewire. One skilled in the art will understand the properties of a workhorse guidewire, including, but not limited to, torque control, trackability, steerability, flexibility, prolapse tendency, radiopacity/visibility, tactile feedback, crossing, and support. Various of these properties are described in Erglis et al, "Tools & Techniques: coronary guidewires", EuroIntervention 2010, 6:1-8, and in Goldberg et al, "Guidewires-Expert Round Table", US Cardiology - Volume 5 Issue 1;2008:5(1):34-38. The entire contents of these articles are hereby incorporated by reference herein. A workhorse guidewire provides balancing of various properties to allow the guidewire to track through vessels, access lesions, cross lesions atraumatically, and provide support for interventional devices that use the guidewire as a track during deployment. For example, torque control and flexibility allow a guidewire to rotate and bend to navigate through vessels, but balance between these two properties is needed-more torque control results in less flexibility, and more flexibility results in less torque control. Certain properties of workhorse guidewires can be quantified, such as flexibility, prolapse, and support. Examples of quantifying such properties in terms of deflection force along the length of a guidewire are shown in FIG. 15. The graph of FIG. 15 was generated by deflecting points along a guidewire at a particular angle and measuring the force with which the guidewire resisted the deflection. Generally, a greater deflection force at a particular point indicates that the guidewire is less flexible at that point but provide more support at that point.

Referring again to FIG. 12, in one embodiment, the four segments 1202-1208 can have an outer diameter between 0.013 and 0.014 inches, and an inner diameter less than 0.011 inches. The core wire 1212 has a diameter of at most 0.007 inches. The four segments 1202-1208 altogether can be referred to as a "housing". The housing can total to approximately 177-180 centimeters in length, which can be substantially coextensive with the core wire 1212 or slightly shorter than the core wire 1212.

One or more of the four housing segments 1202-1208 can be made of a material that is more flexible, deflectable or bendable than the material of the core wire 1212, or less stiff or less rigid than the material of the core wire 1212, or provides more flexibility, deflectability or bendability than the material of the core wire 1212. The core wire 1212 is made of MP35N, L605, Elgiloy, or an alloy of nickel, cobalt, molybdenum and chromium. Generally, 0.014 inch workhorse guidewires could not use MP35N, L605, Elgiloy, or an alloy of nickel, cobalt, molybdenum and chromium, because the material stiffness at 0.014 inches lacks the flexibility and atraumatic characteristics required by a workhouse guidewire to maneuver in the coronary arteries. The disclosed technology provides a guidewire with the characteristics of a 0.014 inch workhorse guidewire while using such materials. The MP35N, L605, Elgiloy, or alloy of nickel, cobalt, molybdenum and chromium are used for the core wire 1212, which can be smaller in diameter than the usual 0.014 inch workhorse guidewire. The core wire 1212 is at most 0.007 inches in diameter. Such a combination of material and diameter size can provide the torque translation and steerability of a 0.014 in workhorse guidewire. To provide the flexibility and atraumatic characteristics of a 0.014 workhorse guidewire, the disclosed technology provides a housing 1202-1208 that is more flexible, deflectable or bendable, or less stiff or less rigid, than the core wire 1212. One or more of the four housing segments 1202-1208 is made of polyemide, nitinol, various types or composition of stainless steel (e.g., 304 & 304 High Tensile), nylon, polyurethane, or PTFE. In one embodiment, the entire housing 1202-1208 can be more flexible, deflectable or bendable, or less stiff or less rigid, than the core wire 1212. In one embodiment, a distal portion of the housing 1202-1208 can be more flexible, deflectable or bendable, or less stiff or less rigid, than the core wire 1212, such as a 40 cm segment of a distal portion of the housing.

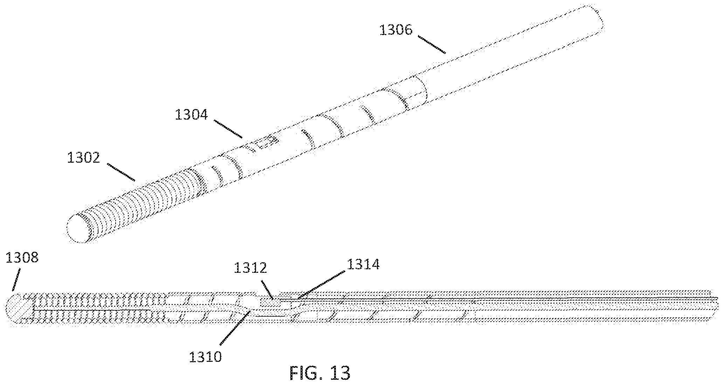

FIG. 13 shows a perspective view and a cross-sectional view of another embodiment of the disclosed monitoring guidewire. The illustrated guidewire housing includes three segments: a distal coil 1302, a slotted tube 1304, and a non-slotted proximal tube 1306. The illustrated embodiment includes one fewer segment than the embodiment of FIG. 12, which can potentially decrease manufacturing difficulties and increase manufacturing yield. The slotted tube 1304 can include a slot pattern that is configured to provide desired properties and characteristics, such as flexibility and/or torque control. The illustrated pattern is merely exemplary and other patterns are contemplated to be within the scope of the disclosed technology. The distal coil 1302 can be configured to provide desired characteristics, such as torsion and/or compression. Referring to the cross-sectional view shown in FIG. 13, the illustrated monitoring guidewire includes an atraumatic tip 1308, a core wire 1310, one or more sensors 1312, and one or more signal wires 1314.

The illustrated monitoring guidewire can provide torque control that approximates that of a 0.014 inch workhorse guidewire. In one embodiment, the three segments 1302-1306 can have an outer diameter between 0.013 and 0.014 inches, and an inner diameter less than 0.011 inches. The core wire 1310 has a diameter of at most 0.007 inches. The three segments 1302-1306 altogether can be referred to as a "housing". The housing can total to approximately 177-180 centimeters in length, which can be substantially coextensive with the core wire 1310 or slightly shorter than the core wire 1310.

One or more of the three housing segments 1302-1306 can be made of a material that is more flexible, deflectable or bendable than the material of the core wire 1310, or less stiff or less rigid than the material of the core wire 1310, or provides more flexibility, deflectability or bendability than the material of the core wire 1310. The core wire 1310 can be made of MP35N, L605, Elgiloy, or an alloy of nickel, cobalt, molybdenum and chromium. The core wire 1310 can be smaller in diameter than the usual 0.014 inch workhorse guidewire. The core wire 1310 is at most 0.007 inches in diameter. Such a combination of material and diameter size can provide the torque translation and steerability of a 0.014 in workhorse guidewire. To provide the flexibility and atraumatic characteristics of a 0.014 workhorse guidewire, the disclosed technology provides a housing 1302-1306 that is more flexible, deflectable or bendable, or less stiff or less rigid, than the core wire 1310. One or more of the three housing segments 1302-1306 is made of polyemide, nitinol, various types or composition of stainless steel (*e.g.,* 304 & 304 High Tensile), nylon, polyurethane, or PTFE. In one embodiment, the entire housing 1302-1306 can be more flexible, deflectable or bendable, or less stiff or less rigid, than the core wire 1310. In one embodiment, a distal portion of the housing 1302-1306 can be more flexible, deflectable or bendable, or less stiff or less rigid, than the core wire 1310, such as a 40 cm segment of a distal portion of the housing.

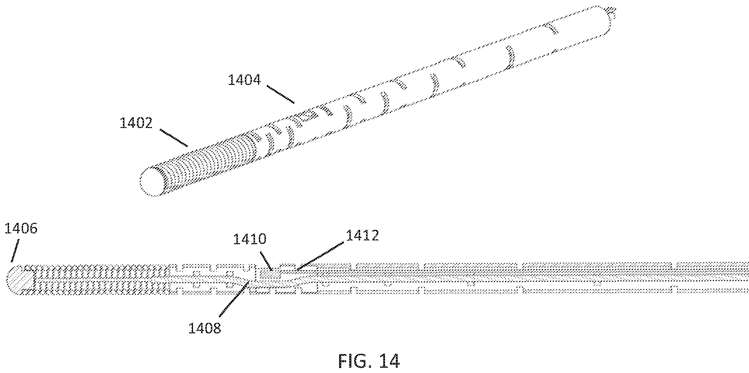

FIG. 14 shows a perspective view and a cross-sectional view of another embodiment of the disclosed monitoring guidewire. The illustrated guidewire housing includes two segments: a distal coil 1402 and a tube 1404 having a slotted distal portion and the remainder being non-slotted. The illustrated embodiment includes one fewer segment than the embodiment of FIG. 13, which can potentially decrease manufacturing difficulties and increase manufacturing yield. The tube 1404 can include a slot pattern that is configured to provide desired properties and characteristics, such as flexibility and/or torque control. The illustrated pattern is merely exemplary and other patterns are contemplated to be within the scope of the disclosed technology. The distal coil 1402 can be configured to provide desired characteristics, such as torsion and/or compression. Referring to the cross-sectional view shown in FIG. 14, the illustrated monitoring guidewire includes an atraumatic tip 1406, a core wire 1408, one or more sensors 1410, and one or more signal wires 1412.

The illustrated monitoring guidewire can provide torque control that approximates that of a 0.014 inch workhorse guidewire. In one embodiment, the two segments 1402-1404 can have an outer diameter between 0.013 and 0.014 inches, and an inner diameter less than 0.011 inches. The core wire 1408 has a diameter of at most 0.007 inches. The two segments 1402-1404 altogether can be referred to as a "housing". The housing can total to approximately 177-180 centimeters in length, which can be substantially coextensive with the core wire 1408 or slightly shorter than the core wire 1408.

One or both of the two housing segments 1402-1404 can be made of a material that is more flexible, deflectable or bendable than the material of the core wire 1408, or less stiff or less rigid than the material of the core wire 1408, or provides more flexibility, deflectability or bendability than the material of the core wire 1408. The core wire 1408 can be made of MP35N, L605, Elgiloy, or an alloy of nickel, cobalt, molybdenum and chromium. The core wire 1408 can be smaller in diameter than the usual 0.014 inch workhorse guidewire. The core wire 1408 is at most 0.007 inches in diameter. Such a combination of material and diameter size can provide the torque translation and steerability of a 0.014 in workhorse guidewire. To provide the flexibility and atraumatic characteristics of a 0.014 workhorse guidewire, the disclosed technology provides a housing 1402-1404 that is more flexible, deflectable or bendable, or less stiff or less rigid, than the core wire 1408. One or both of the two housing segments 1402-1404 is made of polyemide, nitinol, various types or composition of stainless steel (*e.g.,* 304 & 304 High Tensile), nylon, polyurethane, or PTFE. In one embodiment, the entire housing 1402-1404 can be more flexible, deflectable or bendable, or less stiff or less rigid, than the core wire 1408. In one embodiment, a distal portion of the housing 1402-1404 can be more flexible, deflectable or bendable, or less stiff or less rigid, than the core wire 1408, such as a 40 cm segment of a distal portion of the housing.

Various embodiments for a monitoring guidewire have been described above with reference to the drawings. The slots of the slotted tube segments may have different shapes and patterns than those illustrated. The segments may have different dimensions than those illustrated or described.

Various aspects and embodiments of the disclosed technology have been described above. The illustrations and descriptions are merely exemplary and do not limit the scope of the disclosed technology. Even though not illustrated, various embodiments can be combined and are contemplated to fall within the scope of the disclosed technology. Furthermore, although certain features are illustrated as being in a particular location or device, the location and device are merely exemplary, and it is contemplated that various features can be located differently than as illustrated and still be within the scope of the disclosed technology.

The scope of the invention is defined by appended claims 1-6.

## Claims

1. A monitoring guidewire for a medical procedure comprising:

    a core wire (1408) having a first length, wherein the core wire (1408) has a diameter of at most 0.018cm (0.007 inches);
    a pressure sensor (1410) disposed in a distal region of the core wire; and
    a hypotube surrounding the core wire (1408) and having a second length that is less than the first length, the hypotube comprising laser etching along at most a 40cm length of a distal portion of the hypotube,
    wherein the hypotube has an outer diameter between 0.033 and 0.036cm (0.013 and 0.014 inches); and
    wherein the core wire (1408) comprises at least one of: MP35N, L605, Elgiloy, or an alloy including nickel, cobalt, molybdenum and chromium; and wherein the hypotube comprises at least one of: polyemide; nitinol; stainless steel; nylon; polyurethane; PTFE.

2. The monitoring guidewire of claim 1, wherein the core wire (1408) has a length of approximately 180 cm.

3. The monitoring guidewire of claim 2, wherein the hypotube has a length of approximately 177 cm.

4. The monitoring guidewire of claim 1, wherein the laser etching is configured to provide the hypotube with a torque response that approximates a torque response of a 0.036cm (0.014 inch) workhorse guidewire.

5. The monitoring guidewire of claim 4, wherein the hypotube has an inner diameter less than 0.028cm (0.011 inches).

6. The monitoring guidewire of claim 1, wherein the hypotube is more flexible than the core wire (1408) for at most the length of the distal portion of the hypotube having the laser etching.

## Patentansprüche

1. Überwachungsführungsdraht für einen medizinischen Eingriff, umfassend:

    einen Kerndraht (1408), der eine erste Länge aufweist, wobei der Kerndraht (1408) einen Durchmesser von höchstens 0,018 cm (0,007 Zoll) aufweist;
    einen Drucksensor (1410), der in einer distalen Region des Kerndrahts angeordnet ist; und
    ein Hypotube, das den Kerndraht (1408) umgibt und eine zweite Länge aufweist, die geringer als die erste Länge ist, wobei das Hypotube eine Lasergravur entlang höchstens einer Länge von 40 cm eines distalen Abschnitts des Hypotubes umfasst,
    wobei das Hypotube einen Außendurchmesser zwischen 0,033 und 0,036 cm (0,013 und 0,014 Zoll) aufweist und
    wobei der Kerndraht (1408) mindestens eines der folgenden umfasst: MP35N, L605, Elgiloy oder eine Legierung, die Nickel, Kobalt, Molybdän und Chrom enthält;
    und wobei das Hypotube mindestens eines der folgenden umfasst: Polyamid; Nitinol; Edelstahl; Nylon; Polyurethan; PTFE.

2. Überwachungsführungsdraht nach Anspruch 1, wobei der Kerndraht (1408) eine Länge von ungefähr 180 cm aufweist.

3. Überwachungsführungsdraht nach Anspruch 2, wobei das Hypotube eine Länge von ungefähr 177 cm aufweist.

4. Überwachungsführungsdraht nach Anspruch 1, wobei die Lasergravur dazu konfiguriert ist, das Hypotube mit einer Drehmomentübertragung zu versehen, die sich einer Drehmomentübertragung eines Arbeitsführungsdrahts von 0,036 cm (0,014 Zoll) annähert.

5. Überwachungsführungsdraht nach Anspruch 4, wobei das Hypotube einen Innendurchmesser von weniger als 0,028 cm (0,011 Zoll) aufweist.

6. Überwachungsführungsdraht nach Anspruch 1, wobei das Hypotube flexibler als der Kerndraht (1408) für höchstens die Länge des distalen Abschnitts des Hypotubes mit der Lasergravur ist.

## Revendications

1. Fil guide de surveillance pour une intervention médicale comportant :

    un fil central (1408) ayant une première longueur, dans lequel le fil central (1408) a un diamètre de 0,018 cm (0,007 pouce) au plus ;
    un capteur de pression (1410) disposé dans une région distale du fil central ; et
    un hypotube entourant le fil central (1408) et ayant une deuxième longueur qui est inférieure par rapport à la première longueur, l'hypotube comportant une gravure laser le long d'une longueur de 40 cm au plus d'une partie distale de l'hypotube,

dans lequel l'hypotube a un diamètre extérieur entre 0,033 et 0,036 cm (0,013 et 0,014 pouce) ; et

dans lequel le fil central (1408) comporte au moins l'un parmi : MP35N, L605, Elgiloy, ou un alliage comprenant du nickel, du cobalt, du molybdène et du chrome ;

et dans lequel l'hypotube comporte au moins l'un parmi : du polyamide ; du nitinol ; de l'acier inoxydable ; du nylon ; du polyuréthanne ; du PTFE.

2. Fil guide de surveillance selon la revendication 1, dans lequel le fil central (1408) a une longueur d'approximativement 180 cm.

3. Fil guide de surveillance selon la revendication 2, dans lequel l'hypotube a une longueur d'approximativement 177 cm.

4. Fil guide de surveillance selon la revendication 1, dans lequel la gravure laser est configurée pour procurer à l'hypotube une réponse de couple qui correspond approximativement à une réponse de couple d'un fil guide utilitaire de 0,036 cm (0,014 pouce).

5. Fil guide de surveillance selon la revendication 4, dans lequel l'hypotube a un diamètre intérieur inférieur à 0,028 cm (0,011 pouce).

6. Fil guide de surveillance selon la revendication 1, dans lequel l'hypotube est plus flexible que le fil central (1408) pour la longueur au plus de la partie distale de l'hypotube ayant la gravure laser.

Monitoring Guidewire / 102

Portable Display Unit / 104

Protective structure 110

Sensor(s) 108

Core wire 106

Communicaton unit 112

Display screen 114

Battery 116

Memory / storage 118

Communication unit 120

Power management 122

Processor 124

FIG. 1

FIG. 2

Distal Tip of
Interventional Guidewire

IG Sensor(s)

Other Sensors | Temp. Sensor | Pressure Sensor

Portal Display

Display (e.g., LCD) | User Input (e.g., Button)

A/D Converter | µProcessor

(Optional) External Sensor Input

Battery Management System | Battery

(Optional) Output (e.g., USB)

EP 3 313 496 B1

EP 3 313 496 B1

## FIG. 3

302

304

306

FIG. 4

400

408

404

402

410

406

FIG. 5

FIG. 6

FIG. 8

FIG. 7

902  Receive pressure measurements from monitoring guidewire.

904  Designate received pressure measurements as pressure distal to stenosis.

906  Receive pressure measurements from external system.

908  Designate received pressure measurements as pressure proximal to stenosis.

910  Compute simultaneous fractional flow reserve (FFR).

912

Display computed simultaneous FFR and/or a graph of the received pressure measurements

FIG. 9

1002 — Receive pressure measurements from monitoring guidewire.

1004 — Store received pressure measurements as pressure proximal to stenosis.

1006 — Receive pressure measurements from monitoring guidewire.

1008 — Designate received pressure measurements as pressure distal to stenosis.

1010 — Compute push-forward fractional flow reserve (FFR).

1012 — Display computed push-forward FFR and/or a graph of the received and stored pressure measurements

FIG. 10

1102      Receive pressure measurements from monitoring guidewire.

1104      Store received pressure measurements as pressure distal to stenosis.

1106      Receive pressure measurements from monitoring guidewire.

1108      Designate received pressure measurements as pressure proximal to stenosis.

1110      Compute pull-back fractional flow reserve (FFR).

1112      Display computed pull-back FFR and/or a graph of the received and stored pressure measurements

FIG. 11

FIG. 12

FIG. 13

1402

1404

1406

1410

1412

1408

FIG. 14

FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015005648 A1

- US 2013218032 A1

**Non-patent literature cited in the description**

- **ERGLIS et al.** Tools & Techniques: coronary guidewires. *EuroIntervention,* 2010, vol. 6, 1-8

- **GOLDBERG et al.** Guidewires-Expert Round Table. *US Cardiology,* 2008, vol. 5 (1), 34-38